(19) Europäisches Patentamt
European Patent Office
Office européen des brevets

(11) Publication number: **0 529 484 A1**

## (12) EUROPEAN PATENT APPLICATION

(21) Application number: **92114085.1**

(22) Date of filing: **18.08.92**

(30) Priority: **27.08.91 JP 215502/91**

(43) Date of publication of application:
**03.03.93 Bulletin 93/09**

(84) Designated Contracting States:
**BE CH DE ES FR GB IT LI NL SE**

(51) Int. Cl.⁵: **A61K 31/435**

(71) Applicant: **ND NEW DRUG DEVELOPMENT
INSTITUTE INC.
5-2-2, Yayoi-cho, Nakano-ku
Tokyo(JP)**

(72) Inventor: **Takeda, Nobuko
3-18-5, Kugenumasakuragaoka
Fujisawa-shi, Kanagawa-ken(JP)**

(74) Representative: **Wächtershäuser, Günter, Dr.
Tal 29
W-8000 München 2 (DE)**

(54) **Antitussive and expectorant composition.**

(57) An inhalation-type antitussive and expectorant composition comprising a quaternary ammonium-type anti-choline compound as an effective component is disclosed. It exhibits very low toxicity, can suppress coughing, and can advantageously improve difficulty in expectoration.

EP 0 529 484 A1

## BACKGROUND OF THE INVENTION

### Field of the Invention

The present invention relates to an inhalation-type antitussive and expectorant composition comprising a quaternary ammonium-type anti-choline compound as an effective component.

### Description of the Background Art

Difficulty in expectoration is a symptom indicative of a number of respiratory diseases. It not only causes considerable pain but also blocks the airways, inhibiting respiration and inducing the danger of asphyxiation.

Although various expectorants are currently in use, many of them are not properly standardized and the expectoration is not adequately treated principally, because previous studies have not clearly ascertained the physicochemical characteristics of phlegm and its cilia transportation mechanism. Major expectorants currently in use are classified according to the mechanism underlying their action as follows [Clinica, Vol. 18, No. 7, 10-13 (1991)].

### 1. Mucolytic agents

Mucolytic agents fuse phlegm through the chemical action of drugs. Typical examples of mucolytic agents are N-acetylcysteine, methylcysteine hydrochloride, L-ethylcysteine hydrochloride, bromhexine hydrochloride, eprazinone hydrochloride, and the like.

### 2. Mucosa restoration drugs

Mucosa restoration drugs are expectorants which normalize airway conditions and regulate the characteristics of secreted materials so that the materials can more closely match those of physiological airway lubricant. Typical examples are S-carboxymethylcysteine and the like.

### 3. Mucosa lubricating drugs

These drugs lubricate mucosa of the airway, through which phlegm is expelled, by promoting productivity of the lung surfactant. Ambroxisole hydrochloride is a typical example.

Anti-choline compounds have been used as inhalation drugs for ameliorating bronchial asthma, lung emphysema, chronic bronchitis and the like, to expand the bronchi. No antitussive or expectorant activities of anti-choline compounds, however, have been confirmed. Therefore, expectorants have been administered together with anti-choline compounds to patients with these diseases who have expectoration problems.

In view of this situation, the present inventors have undertaken extensive studies, and, as a result, have found that quaternary ammonium-type anti-choline compounds possess a superior antitussive and expectorant activity, leading to the completion of the present invention.

### SUMMARY OF THE INVENTION

Accordingly, an object of the present invention is to provide an inhalation-type antitussive and expectorant composition comprising a quaternary ammonium-type anti-choline compound and a pharmaceutically acceptable carrier.

Other objects, features and advantages of the invention will hereinafter become more readily apparent from the following description.

### BRIEF DESCRIPTION OF THE DRAWINGS

Figure 1 is a graph showing the effect of the antitussive and expectorant composition of the present invention producing improvement in coughing frequency.

Figure 2 is a graph showing the effect of the antitussive and expectorant composition of the present invention producing improvement in coughing intensity.

Figure 3 is a graph showing the effect of the antitussive and expectorant composition of the present invention producing improvement in a feeling of residual phlegm.

Figure 4 is a graph showing the effect of the antitussive and expectorant composition of the present invention producing improvement in phlegm elimination.

DETAILED DESCRIPTION OF THE INVENTION AND PREFERRED EMBODIMENTS

Given as examples of quaternary ammonium-type anti-choline compounds, which constitute the effective components of the antitussive and expectorant composition of the present invention, are flutropium bromide, ipratropium bromide, oxytropium bromide, atropine methonitrate, and the like. Among them, flutropium bromide, having the chemical name "8(r)-8-(2-fluoroethyl)-3α-hydroxy-1αH,5αH-tropanium bromide, benzilate hydrate" is preferred.

Flutropium bromide has already been used as an inhalation drug for asthma or bronchial diseases. Its toxicity is extremely low, with $LD_{50}$ through oral administration being 930 mg/kg in mouse and 2,900 mg/kg in rat.

The antitussive and expectorant composition of the present invention can be prepared by processing a quaternary ammonium-type anti-choline compound (hereinafter simply referred to as anti-choline compound) into an inhalation-type preparation according to a conventional method. One example of such preparations is a blend of an anti-choline compound and an excipient, pulverized into fine powder having a particle size of 10 μm or smaller, and filled in hard capsules or suitable containers. The powder may be dispersed by a propellant or compressed air for inhalation. Alternatively, the powder may directly be dissolved or dispersed into a propellant using a dissolving adjuvant or a dispersant to formulate it into an aerosol for inhalation.

As excipients used for the above preparation, lactose, sorbitol, mannitol, starch, microcrystalline cellulose, and the like are given. Nonionic surfactants which can be orally administered, soybean lecithin, yolk lecithin, and the like are given as examples of dissolving adjuvants and dispersants. Liquefied fluorinated hydrocarbons (Freons [R]),carbon dioxide gas, nitrogen gas, or the like can be used as a propellant.

A dose of the antitussive and expectorant composition of the present invention varies depending on the age and symptom of the patients. For adults, 1-3 inhalations (30-90 μg as anti-choline compound) at one time, 3-4 times a day, by spray inhalation into the oral cavity are preferable.

The antitussive and expectorant composition of the present invention exhibits very low toxicity, can suppress coughing, and can advantageously improve difficulty in expectoration.

Other features of the invention will become apparent in the course of the following description of the exemplary embodiments which are given for illustration of the invention and are not intended to be limiting thereof.

EXAMPLES

Example 1

0.05 part by weight of flutropium bromide, pulverized into fine powder having a particle size of 10 μm or smaller, and 0.1 part by weight of soybean lecithin were placed in a pressure vessel equipped with a cooling device and a stirrer. Then, 100 parts by weight of a propellant (Freon 12 [R]/Freon 11 [R] =70/30) was charged into the vessel under pressure. The content was cooled below -50°C and filled into an aluminum container while stirring. A quantitative propellant dispenser, 30 μg of the essential compounds per spout, was fitted to the container.

Example 2

The same procedure as in Example 1 was followed, except that Polysorbate 80 (a polyoxyethylene ether of anhydrous sorbitol, partially esterified with oleic acid; Pharmacopeia of Japan) was used instead of the soybean lecithin.

Example 3

0.2 Part by weight of flutropium bromide was homogeneously dispersed in 10 parts by weight, out of 100 parts by weight, of lactose. The remaining portion of lactose was gradually added to prepare a uniform mixture. The mixture was pulverized into fine powder having a particle size of 10 μm or smaller and filled into No. 2 hard gelatin capsules, each containing 30 mg of the powder.

3

Example 4

Capsules were prepared in the same manner as in Example 3 by using starch instead of lactose.

Test Example 1

The antitussive and expectorant composition prepared in Example 1 was administered to 40 patients suffering from coughing and difficulty in expectoration, 4 times a day, 2 inhalations at one time, to observe their improvement after 1 week and 2 weeks, respectively. The evaluation was made based on the following parameters.
(1) Frequency of coughing
(2) Degree of coughing
(3) A feeling of residual phlegm
(4) Elimination of phlegm

General Improvement

The number of patients showing improvement by degree in parameters 1-4 is shown in Table 1.

TABLE 1

|  | After 1 week | | After 2 weeks (Final) | |
| --- | --- | --- | --- | --- |
|  | Number of patients | Incremental percentage | Number of patients | Incremental percentage |
| Improvement was remarkable | 15 | 37.5 | 20 | 50.0 |
| Improvement was average | 18 | 82.5 | 12 | 80.0 |
| Improvement was slight | 1 | 85.0 | 1 | 82.5 |
| No improvement was seen | 4 | 95.0 | 4 | 92.5 |
| Symptoms exacerbated | 2 | 100.0 | 3 | 100.0 |
| Total | 40 |  | 40 |  |

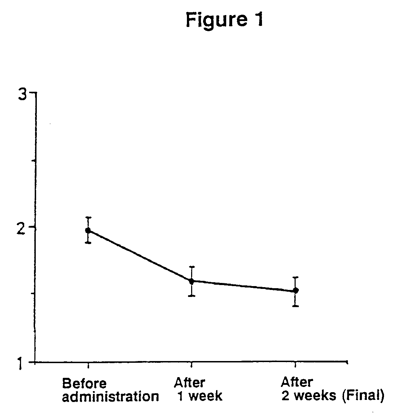

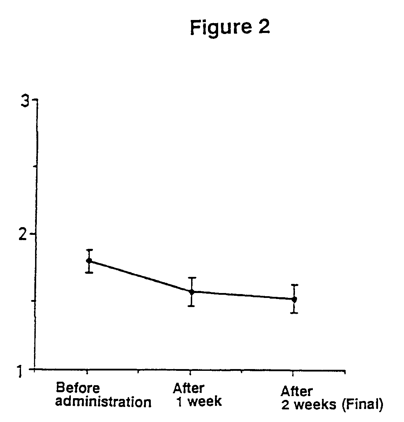

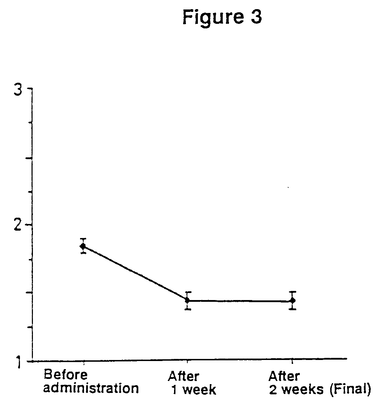

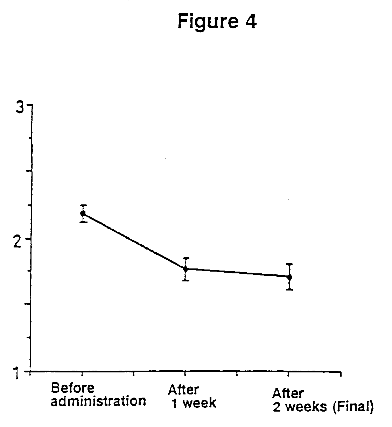

Before administration and at 1 and 2 weeks after administration, the subjects were evaluated relative to each of the above 4 parameters and graded into 3 classes as follows.
(1) Frequency of coughing
1:      infrequent
2:      frequent
3:      very frequent
(2) Degree of coughing
1:      weak
2:      strong
3:      very strong
(3) A feeling of residual phlegm
1:      weak
2:      strong
3:      very strong
(4) Elimination of phlegm.
1:      easily eliminated
2:      rather difficult to eliminate
3:      very difficult to eliminate
Mean values of the grades of the 40 subjects at each time of the evaluations were calculated. The results are shown in Figures 1-4.

Side effects and accompanying symptoms

Presence or absence of side effects and accompanying symptoms during the test was determined. If present, they were grouped into 3 classes, i.e., (i) side effects and symptoms related to the administration,

4

(ii) side effects and symptoms not related to the administration, and (iii) side effects and symptoms which could not be ascertained as to whether or not they were related. The results are shown in Table 2.

## TABLE 2

|  | Number of subjects | % |
|---|---|---|
| No side effects nor symptoms were detected | 40 | 100 |
| Side effects or symptoms were observed | | |
| Related | 0 | 0.0 |
| Not related | 0 | 0.0 |
| Could not ascertain | 0 | 0.0 |
| Total | 40 | |

Obviously, numerous modifications and variations of the present invention are possible in light of the above teachings. It is therefore to be understood that within the scope of the appended claims, the invention may be practiced otherwise than as specifically described herein.

## Claims

1. An inhalation-type antitussive and expectorant composition comprising a quaternary ammonium-type anti-choline compound and a pharmaceutically acceptable carrier.

2. The composition according to Claim 1, wherein said quaternary ammonium-type anti-choline compound is flutropium bromide.

## Figure 1

# Figure 2

## Figure 3

## Figure 4

European Patent
Office

**PARTIAL EUROPEAN SEARCH REPORT**
which under Rule 45 of the European Patent Convention
shall be considered, for the purposes of subsequent
proceedings, as the European search report

Application Number

EP 92 11 4085

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 705 213 (CHIESI FARMACEUTICI S.p.A.) <br> * Claim 12 * <br> --- | 1-2 | A 61 K 31/435 |
| X | JOURNAL OF ASTHMA, vol. 18, no. 1, January 1981, pages 43-46; C.J. FALLIERS: "Conbined vs. sequential vs. single-entity therapy" <br> * Page 46 * <br> --- | 1-2 | |
| X | DE-A-2 540 633 (C.H. BOEHRINGER SOHN) <br> * Page 8 * <br> --- | 1-2 | |
| X | EUR. J. RESP. DIS., vol. 71, suppl. 153, 1987, pages 173-181; R.S. IRWIN et al.: "The effects of drugs on cough" <br> * Abstract * <br> ---        -/- | 1-2 | |

TECHNICAL FIELDS
SEARCHED (Int. Cl.5)

A 61 K 31/00

## INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet -C-

| Place of search | Date of completion of the search | Examiner |
|---|---|---|
| THE HAGUE | 11-11-1992 | THEUNS H.G. |

European Patent
Office

**PARTIAL EUROPEAN SEARCH REPORT**

Application Number

EP   92 11 4085

| | DOCUMENTS CONSIDERED TO BE RELEVANT | | CLASSIFICATION OF THE APPLICATION (Int. Cl.5) |
|---|---|---|---|
| Category | Citation of document with indication, where appropriate, of relevant passages | Relevant to claim | |
| X | CHEST, vol. 86, no. 3, September 1984, pages 387-393; M.A. GHAFOURI et al.: "Sputum changes associated with the use of ipratropium bromide" * Entire document * --- | 1-2 | |
| X | EUROPEAN JOURNAL OF RESPIRATORY DISEASES, vol. 64, suppl. 128, part 1, 1983, pages 304-317; D. PAVIA et al.: "Drug effects on mucociliary function" * Page 310 * --- | 1-2 | |
| X | CHEST, vol. 93, no. 6, June 1988, pages 1186-1189; R. LOWRY et al.: "Antitussive properties of inhaled bronchodilators on induced cough" * Entire document * --- | 1-2 | **TECHNICAL FIELDS SEARCHED (Int. Cl.5)** |
| X | THE SAISHIN-IGAKU, vol. 45, no. 1, 1990, pages 95-100; J. KABE: "Pharmacological management of obstructive lung disease" * Page 97; summary * --- | 1-2 | |
| X | DIALOG INFORMATION SERVICES, file 155, medline 1966-1992/Dec., accession no. 06432616; R. LOWRY et al.: "Inhibition of artificially induced cough in man by bronchodilators", & BR. J. CLIN. PHARMACOL., OCTOBER 1987, 24(4), P. 503-10 * Abstract * ----- | 1-2 | |

EPO FORM 1503 03.82 (P0410)

EP 92 11 4085

-C-

In view of the definition of compounds by means of
their pharmacological activity instead of by
structural parameters, the search was limited to
the compounds identified in the description
(Art. 84 EPC; Guidelines, BII,7  last sentence and
BIII, 3.7).